# EUROPEAN PATENT SPECIFICATION

(11) **EP 2 251 011 B1**
(45) Date of publication and mention of the grant of the patent: **04.04.2012**
(21) Application number: 09712557.9
(22) Date of filing: 20.02.2009
(51) Int. Cl.: A61K 31/495, A61K 9/20, A61K 47/02, A61K 47/32, A61K 47/36, A61K 47/38, A61P 25/18, A61P 43/00, A61K 47/26

(54) **SOLID PREPARATION FOR ORAL ADMINISTRATION**
FESTE ZUBEREITUNG ZUR ORALEN VERABREICHUNG
PRÉPARATION SOLIDE POUR ADMINISTRATION ORALE

(30) Priority: 21.02.2008 JP 2008040696; 05.11.2008 JP 2008284685
(43) Date of publication of application: 17.11.2010
(73) Proprietor: Richter Gedeon Nyrt., 1103 Budapest, (HU)
(72) Inventor: OOBAYASHI, Yasuaki, Osaka-shi Osaka 541-8505 (JP); OOKAWA, Akiko, Osaka-shi Osaka 541-8505 (JP); HADAMA, Atsuko, Tokyo 111-0042 (JP)
(74) Representative: HOFFMANN EITLE
(86) International application number: PCT/JP2009/053039
(87) International publication number: WO 2009/104739

(56) References cited:
- WO-A1-2005/012266
- WO-A1-2010/009309

## Description

### TECHNICAL FIELD

The present invention relates to a solid preparation for oral administration of cariprazine hydrochloride useful as a medicament for treating diseases such as schizophrenia.

### BACKGROUND ART

It is described in the patent document 1 listed below that cariprazine hydrochloride, of the following formula is a D3/ D2 receptor antagonist and is useful for a medicament for treating diseases such as schizophrenia. 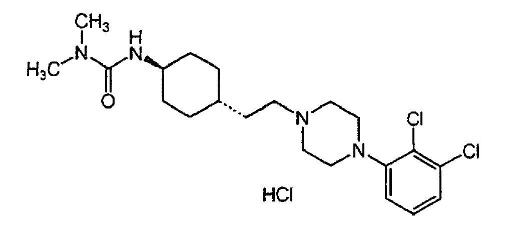 The following prescription example of a tablet containing a compound of the formula (I) comprising cariprazine hydrochloride as an active ingredient as well as cyclodextrin is disclosed on page 27 in the above publication.

| | |
|---|---|
| "Compound of formula (I) | 1-40 mg |
| Diluent/ Filler (it may be cyclodextrin) | 50-250 mg |
| Binder | 5-25 mg |
| Disintegrant (it may be cyclodextrin) | 5-50 mg |
| Lubricant | 1-5 mg |
| Cyclodextrin | 1-100 mg |

Diluent: microcrystalline cellulose, lactose, starch and the like.
Binder: polyvinylpyrrolidone, hydroxypropyl methylcellulose and the like.
Disintegrant: sodium starch glycolate, crospovidone and the like.
Lubricant: magnesium stearate, sodium stearyl fumarate and the like."

It is generally known that a chemically unstable compound can be stabilized by adding cyclodextrin to a preparation. In addition, cyclodextrin has effects such as an improvement in water solubility of a medicinal compound, an improvement in bioavailability and reduction of bitterness (masking), and therefore, is applied for a medicinal product.

[Patent document 1] WO2005/012266

### DISCLOSURE OF INVENTION

### PROBLEM TO BE SOLVED

Although cyclodextrin is expected to have a stabilizing effect, in a case where cyclodextrin is used as an additive for medicament, due to inclusion of the drug, an influence to disposition and medicinal effect, and an influence to other drugs, used in combination are unclear. In view of these points, a solid preparation for oral administration which does not contain cyclodextrin and can stably store cariprazine hydrochloride is desired. The object of the present invention is to provide said solid preparation for oral administration.

### MEANS FOR SOLVING PROBLEM

The present inventors earnestly studied as to various solid preparations containing cariprazine hydrochloride. As a result, they have found the following facts and completed the present invention. When mannitol or anhydrous calcium hydrogen phosphate was contained as a part of an excipient in a preparation as well as when only crystalline cellulose was used as an excipient, a large amount of related substances was produced. However, when lactose was used as a main excipient, cariprazine hydrochloride was stably stored without adding cyclodextrin.

That is, the present invention is provided as follows.
(1) A solid preparation for oral administration which uses lactose as a main excipient and comprises cariprazine hydrochloride and which does not contain cyclodextrin,
(2) The solid preparation according to item (1) wherein an excipient other than lactose is crystalline cellulose and/or starch.
(3) The solid preparation according to item (2) wherein with respect to total amount of excipient, 50-100 % by weight is lactose, 0-50 % by weight is crystalline cellulose and 0-35 % by weight is starch.
(4) The solid preparation according to item (2) wherein with respect to total amount of excipient, 60-100 % by weight is lactose, 0-29 % by weight is crystalline cellulose and 0-11 % by weight is starch.
(5) The solid preparation according to item (2) wherein with respect to total amount of excipient, 70-100 % by weight is lactose, 0-25 % by weight is crystalline cellulose and 0-5% by weight is starch.
(6) The solid preparation according to item (2) wherein with respect to total amount of excipient, 80-95 % by weight is lactose, 5-20 % by weight is crystalline cellulose and no starch is contained in the solid preparation.
(7) The solid preparation according to any one of items (1)-(6) wherein at least one binder selected from hydroxypropyl cellulose, hydroxypropyl methylcellulose, ethylcellulose, methylcellulose, povidone and polyvinyl alcohol is used.
(8) The solid preparation according to any one of items (1)-(7) wherein at least one disintegrant selected from sodium starch glycolate, croscarmellose sodium, low substituted hydroxypropyl cellulose, powdered agar and crospovidone is used.
(9) The solid preparation according to any one of items (1)-(8), wherein the preparation is prepared by blending cariprazine hydrochloride, an excipient, a binder, and, if needed, a disintegrant; granulating the mixture; blending a disintegrant and a lubricant to the granule; and then compacting the granule.

### EFFECT OF THE INVENTION

A solid preparation for oral administration of the present invention can be stably stored without adding cyclodextrin.

### BRIEF DESCRIPTION OF DRAWINGS

[Figure 1] Time-dependent change of the related substances in the tablets prepared in Example 4 is shown.
[Figure 2] Time-dependent change of the related substances in the tablets prepared in Example 8 is shown.
[Figure 3] Time-dependent change of the related substances in the tablets prepared in Example 9 is shown.
[Figure 4] Time-dependent change of the related substances in the tablets prepared in Example 10 is shown.
[Figure 5] Time-dependent hardness change of the tablets prepared in Examples 18 and 20 is shown.

### BEST MODE FOR CARRYING OUT THE INVENTION

In the present specification, an amount of cariprazine hydrochloride in each tablet is normally 0.01-70 % by weight, preferably 0.1-50 % by weight, more preferably 0.4-10 % by weight. Main excipient is an excipient which is contained by an amount of equal to or more than 50 % by weight of the total amount of excipient, and lactose is used as the main excipient in the solid preparation of the present invention. Other excipients include crystalline cellulose and starch (for example, corn starch, potato starch, wheat starch, rice starch, partly pregelatinized starch and porous starch) and the like. Crystalline cellulose and starch have water retentivity, and therefore by adding them as part of excipient, lot-to-lot variation of the solid preparation during wet granulation can be reduced and the filling property into a mortar of a tableting machine is improved. The preferable excipients include (a) an excipient wherein with respect to total amount of excipient, 50-100 % by weight is lactose, 0-50 % by weight is crystalline cellulose and 0-35 % by weight is starch; (b) an excipient wherein with respect to total amount of excipient, 60-100 % by weight is lactose, 0-29 % by weight is crystalline cellulose and 0-11 % by weight is starch; (c) an excipient wherein with respect to total amount of excipient, 70-100 % by weight is lactose, 0-25 % by weight is crystalline cellulose and 0-5 % by weight is starch; and (d) an excipient wherein with respect to total amount of excipient, 80-95 % by weight is lactose, 5-20 % by weight is crystalline cellulose and no starch is contained. In addition, the decrease of hardness of the solid preparation can be reduced and cariprazine hydrochloride can be stably stored whatever the humidity is low or high by not using starch or using a low amount of starch. These effects are the preferable effects. Each tablet can contain 5-99.9 % by weight, preferably 80-95 % by weight of the excipient.

The binders include hydroxypropyl cellulose, hydroxypropyl methylcellulose, ethylcellulose, methylcellulose, polyvinylpyrrolidone, povidone, polyvinyl alcohol, gum arabic powder, gelatine, pullulan and the like. The binder such as hydroxypropyl cellulose is preferable. Each tablet can contain 0.5-10 % by weight, preferably 1-5 % by weight of the binder.
Carmellose calcium, croscarmellose sodium, sodium starch glycolate, crospovidone, low substituted hydroxypropyl cellulose, powdered agar and the like are used as the disintegrant. The disintegrants such as sodium starch glycolate, croscarmellose sodium and low substituted hydroxypropyl cellulose are preferable. Each tablet can contain 0.1-15 % by weight, preferably 1-5 % by weight of the disintegrant.

In addition, the solid preparation of the present invention may contain appropriate amount of a flavor, a lubricant, a coloring agent and the like, or various additives which are commonly used for preparing a formulation unless any trouble in the effect of the present invention arises. The lubricants include magnesium stearate, calcium stearate, sucrose fatty acid ester, polyethylene glycol, talc, stearic acid, sodium stearyl fumarate and the like. The coloring agents include the food colors such as food yellow no. 5, food red no. 2, food blue no. 2, food lake colors, iron sesquioxide and the like. Further, when the solid preparation of the present invention is prepared, a coating mixture may be used by a well-known method with the purpose of, for example, further masking of a taste and an odor, and preparation of an enteric formulation or a sustained-release formulation after coating a particle core with the active ingredient, an additives and the like.

The solid preparations of the present invention include, for example, a tablet, a capsule, a granule and a pill. The tablet is preferable. A shape of the tablet is not specified and the tablet may be an uncoated tablet with a shape such as round, oval, oblong and a coated tablet thereof. In addition, the solid preparation of the present invention may be a grouping tablet which is tableted after mixing two or more types of granules, a multilayer tablet such as a double-layer tablet and a triple-layer tablet, a dry-coated tablet as well as a presscoating tablet.

The solid preparations of the present invention can be prepared by, for example, wet granulating cariprazine hydrochloride, an excipient, a binder and/or a disintegrant with water or a binder solution using a machine such as a high speed mixer granulator, a fluidized-bed granulator dryer, a centrifugal tumbling fluidized-bed granulator coating machine or a kneading machine; blending or spraying a lubricant to the granules; and then subjecting to compression molding. Alternatively, the solid preparations of the present invention can be prepared by dry granulating cariprazine hydrochloride, an excipient and/or a binder (a disintegrant may be further contained) using a machine such as a roller compacter; blending or spraying a disintegrant (a lubricant may be further contained) to the granules; and then subjecting to compression molding.

### EXAMPLES

The present invention is illustrated in more details by the following Examples.
The following machines were used in the Examples.
High speed mixer granulator: FM-VG-10 or FM-VG-25 made by Powrex Corporation;
Tumbling fluidized-bed granulator dryer: MP-01 made by Powrex Corporation;
Fluidized-bed granulator dryer: FLO-5/2SJ made by Freund Corporation or MP-01 made by Powrex Corporation;
Particle size regulator: QC-197S (0.991 mm circular hole screen) made by Powrex Corporation;
V-Blender: VM-10 made by Fuji Paudal Co., Ltd. or TCV-20 made by Tokuju Corporation; and
Tableting machine: AQUA0518SS2AII or VIRG0518SS2AZ made by Kikusui Seisakusho Ltd.
The units of tableting pressure are shown in the Tables as the unit displayed on machines (AQUA: kgf and VIRG: kN).

### Example 1

Each component shown in the column of "dry powder" in Table 1 was put in a high speed mixer granulator, blended, and then, granulated with water. The granule was dried in a tumbling fluidized-bed granulator dryer to give the dry powder. The resulting dry powder was blended with each component shown in the column of "bulk powder mix for tablets" in Table 1 by a V-Blender to give the bulk powder for tablets. This bulk powder for tablets was compacted (tablet diameter: 7 mm; a radius of curvature: 10 mm) to prepare tablets (120 mg each), each containing 0.5 mg of cariprazine hydrochloride.

### Example 2

Each component shown in Table 1 was blended and tablets (120 mg each), each containing 2.5 mg of cariprazine hydrochloride, were prepared in accordance with the method described in Example 1 (the high speed mixer granulator method).

### Example 3

Each component shown in the column of "dry powder" in Table 1 (except a binder) was put in a high speed mixer granulator and blended. This mixture was put in a fluidized-bed granulator dryer to fluidize, granulated in the fluidized-bed with a binder which was prepared by amount shown in Table 1, and then, dried in the bed to give the dry powder. A size of the dry powder was regulated in a particle size regulator and the resulting regulated powder was blended with each component shown in the column of "bulk powder mix for tablets" in Table 1 by a V-Blender to give a bulk powder for tablets. This bulk powder for tablets was compacted (tablet diameter: 7 mm; a radius of curvature: 10 mm) to prepare tablets (120 mg each), each containing 0.5 mg of cariprazine hydrochloride.

### Example 4

Components shown in Table 1 were blended and tablets (120 mg each), each containing 0.5 mg of cariprazine hydrochloride, were prepared in accordance with the method described in Example 3 (the fluidized-bed granulation method).

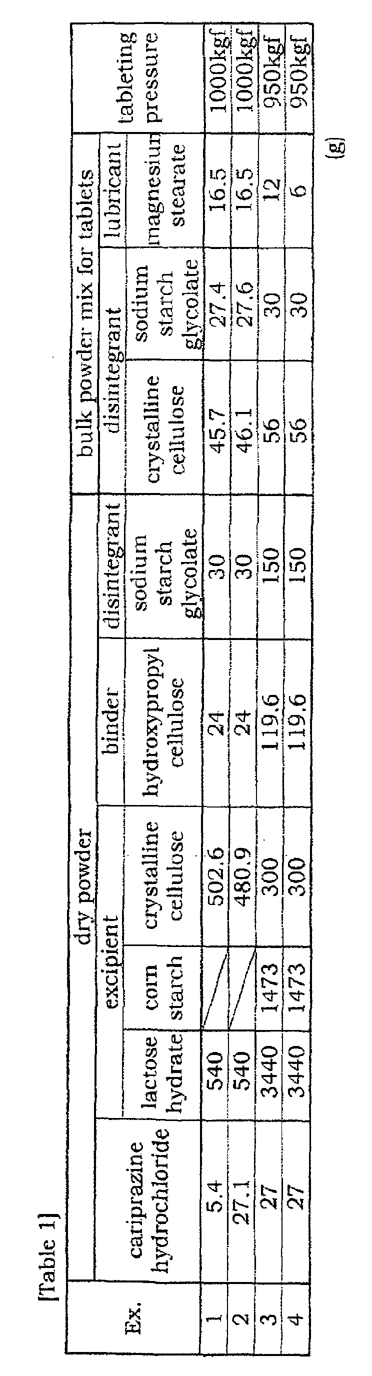

### Example 5

Each component shown in the column of "dry powder" in Table 2 (except a binder) was put in a high speed mixer granulator and blended. This mixture was put in a fluidized-bed granulator dryer to fluidize, granulated in the fluidized-bed with a binder which was prepared by amount shown in Table 2, and then, dried in the bed to give the dry powder. A size of the dry powder was regulated in a particle size regulator and the resulting regulated powder was blended with each component shown in the column of "bulk powder mix for tablets" in Table 1 in a V-Blender to give the bulk powder for tablets. This bulk powder for tablets was compacted (tablet diameter: 7 mm; flat with bevel edge) to prepare tablets (120 mg each), each containing 0.5 mg of cariprazine hydrochloride.

### Example 6 - Example 19

Components shown in Table 2 were blended and tablets were prepared in accordance with the method described in Example 5 (the fluidized-bed granulation method). The weight of each tablet was 120 mg and amount of each component was shown in Table 2.

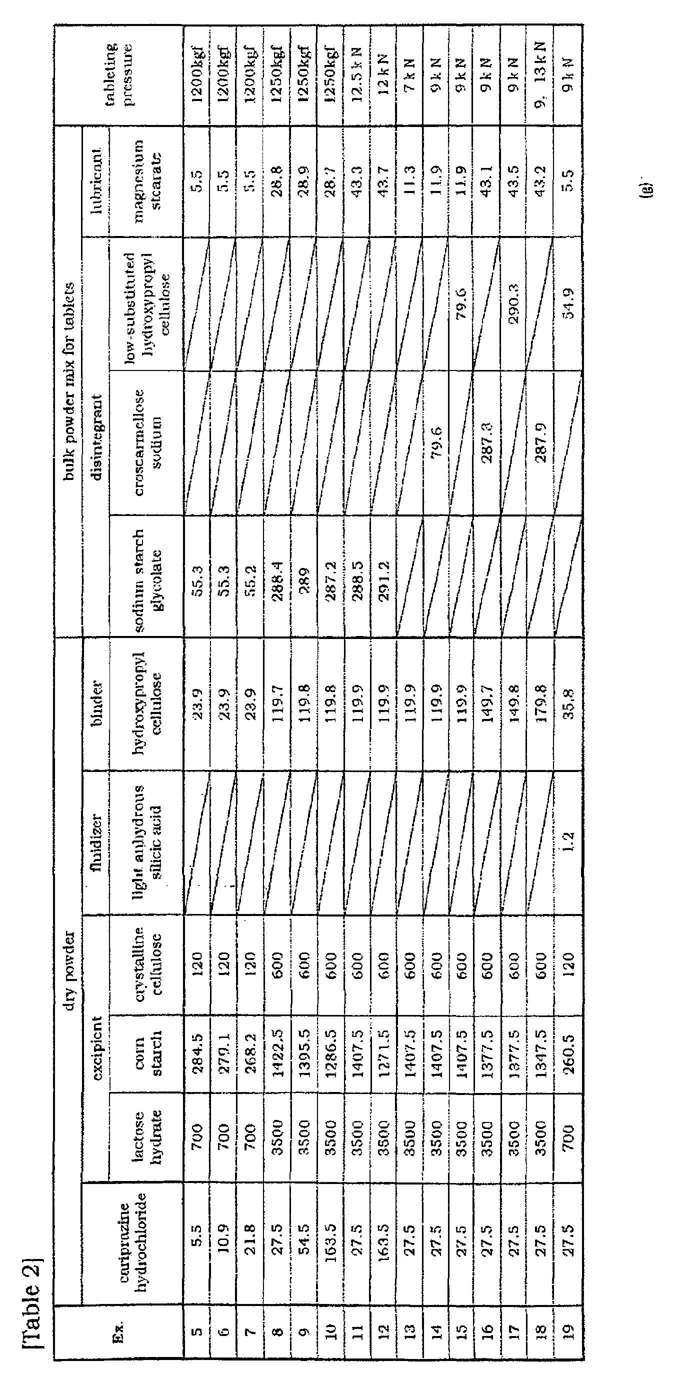

### Example 20 - Example 33

Components shown in Table 3 were blended and tablets were prepared in accordance with the method described in Example 5 (the fluidized-bed granulation method). The weight of each tablet was 120 mg and amount of each component was shown in Table 3.

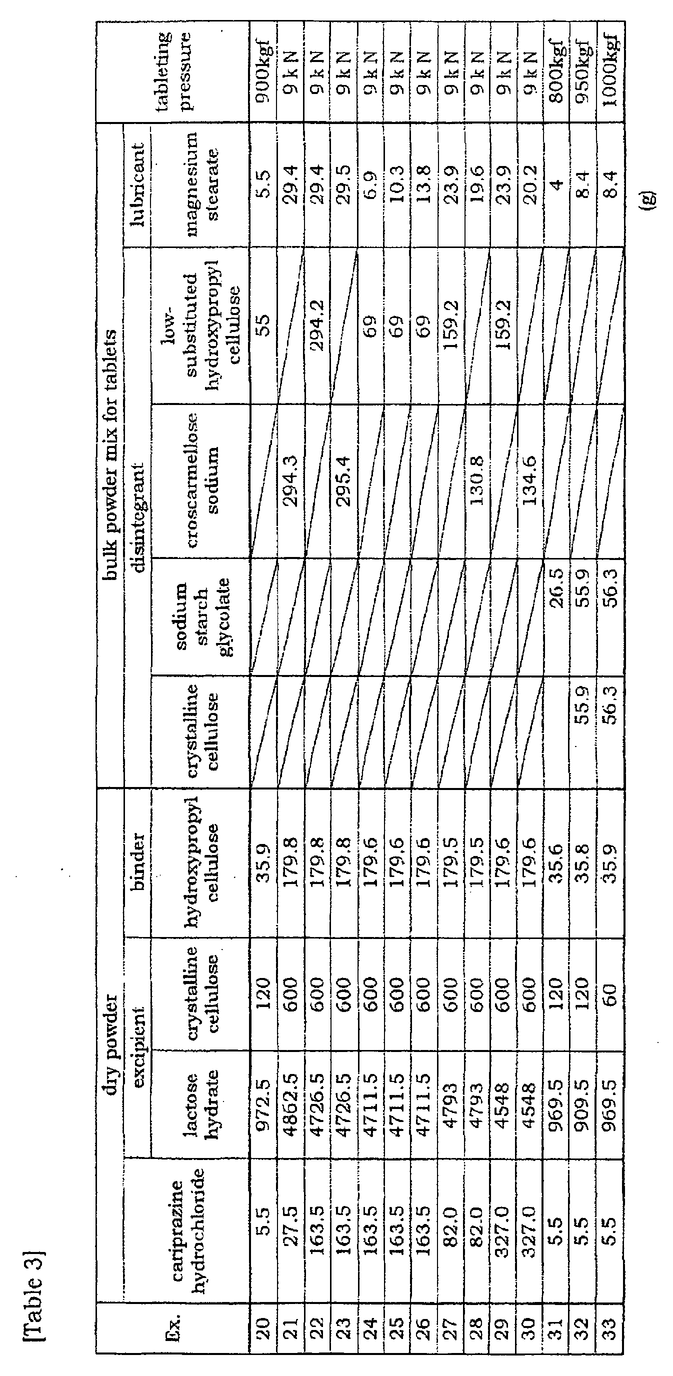

### Comparative Example 1

Each component shown in the column of "dry powder" in Table 4 excepting a binder was put in a laboratory universal powder and granular material processing equipment (Mechanomill made by Okada Seiko Co., Ltd.) and blended. This mixture was put in a fluidized-bed granulator dryer to fluidize, granulated in the fluidized-bed with a binder which was prepared by amount shown in Table 4, and then, dried in the bed to give the dry powder. A size of the dry powder was regulated in a particle size regulator and the resulting regulated powder was mixed with each component shown in the column of "bulk powder mix for tablets" in Table 4 in a V-Blender to give the bulk powder for tablets. This bulk powder for tablets was compacted (tablet diameter: 7 mm; flat with bevel edge) to prepare tablets (120 mg each), each containing 0.5 mg of cariprazine hydrochloride. An amount of each component and a method for preparation were in accordance with the those of Example 5 except that lactose hydrate was exchanged to D-mannitol.

### Comparative Example 2

Components shown in Table 4 were put in a laboratory universal powder and granular material processing equipment, and blended to give the bulk powder mix for tablets. This bulk powder for tablets was compacted (tablet diameter: 7 mm; flat with bevel edge) to prepare tablets (120 mg each), each containing 0.5 mg of cariprazine hydrochloride.

### Comparative Example 3

Components shown in Table 4 were put in a laboratory universal powder and granular material processing equipment, and blended to give the bulk powder mix for tablets. This bulk powder for tablets was compacted (tablet diameter: 7 mm; flat with bevel edge) to prepare tablets (120 mg each), each containing 0.5 mg of cariprazine hydrochloride.

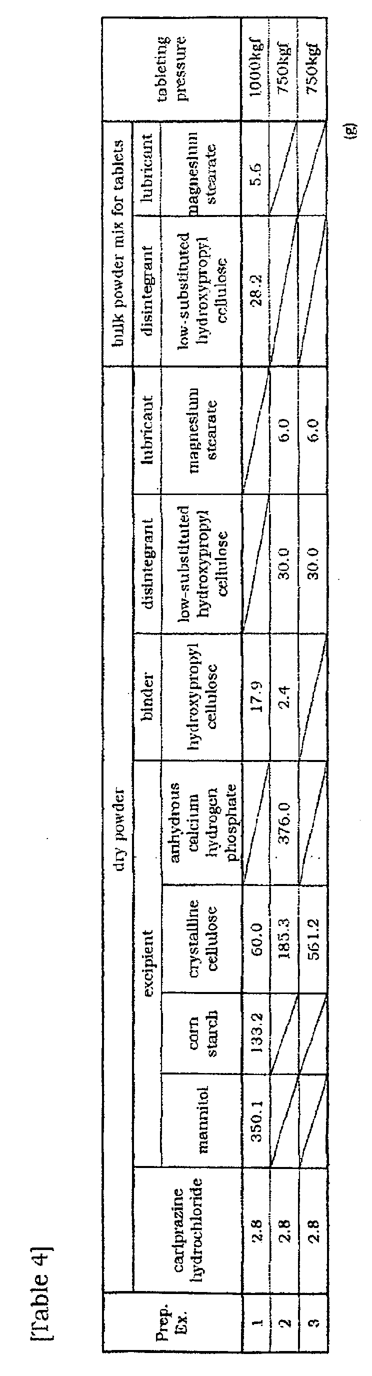

The stabilities of the tablets obtained in the above Examples in various package presentations were evaluated.

### Experimental Example 1: Stability Test

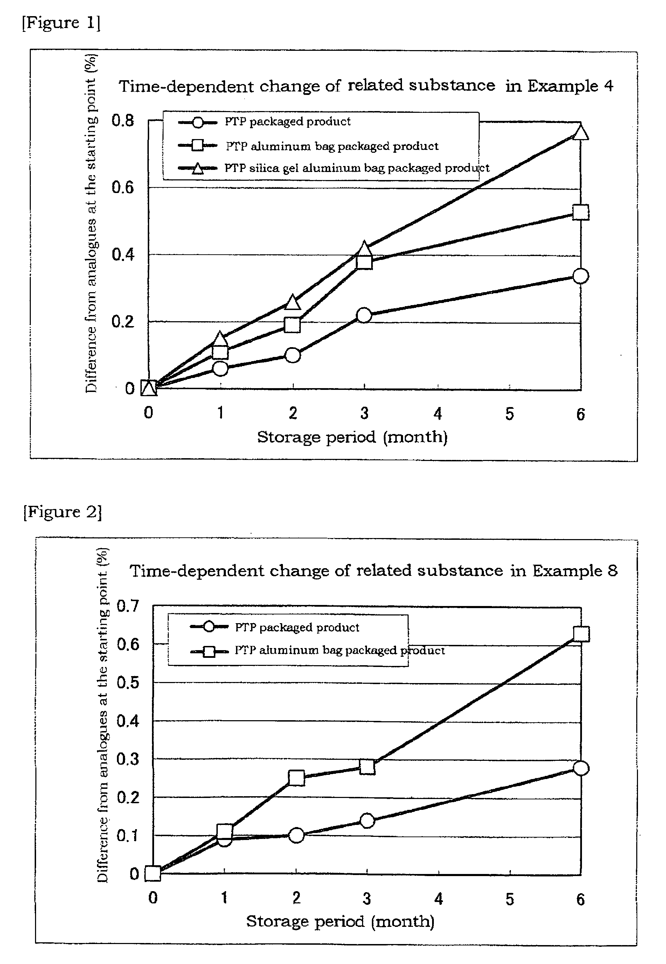

The tablets obtained in Example 4 were packaged in a PTP (PVC) blister package (PVC molded sheet: Daiwakasei Industry Co., Ltd., aluminum foil for PVC: Daiwakasei Industry Co., Ltd., ten tablets per sheet) to give the PTP packaged product. In addition, as a comparative example in a high moisture-proof packaging, ten sheets of this PTP packaged product were put in an aluminum gusset bag (Okada Shigyo Co., Ltd.) and the bag was subjected to heat-sealing by a heat-sealer (Fujiimpulse Co., Ltd) to give the PTP aluminum bag packaged product. Ten sheets of the PTP packaged product and silica-gel (Shin-etsukasei Industriy Co., Ltd.; Silica gel Sanpo 1 g) were put in an aluminum gusset bag (Okada Shigyo Co., Ltd.) and the bag was subjected to heat-sealing by a heat-sealer (Fujiimpulse Co., Ltd) to give a PTP silica gel aluminum bag packaged product. The stability tests of the PTP packaged product, the PTP aluminum bag packaged product and the PTP silica gel aluminum bag packaged product were conducted by storing in a constant temperature and humidity room (the room temperature was 40 °C; the relative humidity was 75 %). The storage periods were 1, 2, 3 and 6 months in all package presentations. After each period, each content was taken out from the package and the related substances were confirmed by a high-performance liquid chromatography to observe the time-dependent difference of the related substances in the tablet between the starting point of the storage and each point after storage (Fig. 1).

### Experimental Example 2: Stability Test

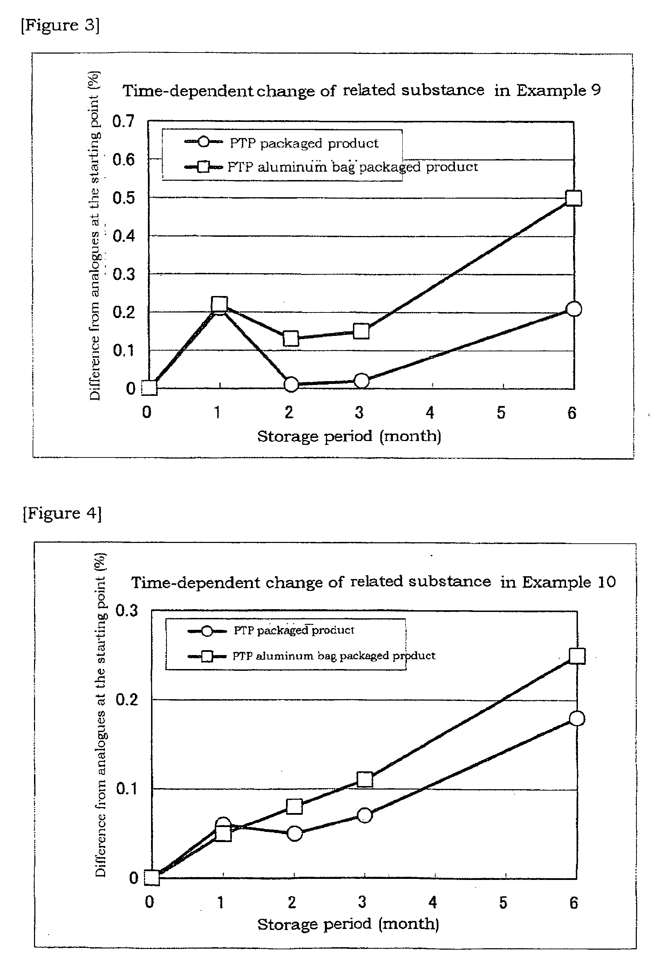

The tablets obtained in Example 8, Example 9 and Example 10 were packaged in accordance with Experimental Example 1 to give the PTP packaged products and the PTP aluminum bag packaged products. The stability tests of the PTP packaged products and the PTP aluminum bag packaged products were conducted by storing in a constant temperature and humidity room (the room temperature was 40 °C; the relative humidity was 75 %). The storage periods were 1, 2, 3 and 6 months in all package presentations. After each period, each content was taken out from the package and the related substances were confirmed by a high-performance liquid chromatography to observe the time-dependent difference of the related substances in the tablet between the starting point of the storage and each point after storage (Fig. 2, Fig. 3 and Fig. 4).
As shown in Fig. 1, Fig. 2, Fig. 3 and Fig. 4 derived from Experimental Examples 1 and 2, every tablet obtained in Examples 4, 8, 9 and 10 hardly produced related substances, and therefore, these tablets have excellent storage stability. In addition, there was a tendency that the package presentation having higher moisture-proof property produced more related substances.

### Experimental Example 3: Stability Test

The stability tests of the teblets obtained in Comparative Examples 1, 2 and 3 as well as Examples 12, 14 and 15 were conducted by storing their PTP packaged products in a constant temperature chamber (the room temperature was 60 °C) . The storage period was 2 months and the related substances were confirmed by a high-performance liquid chromatography. Produced amount of the related substances under the condition of the storage for 2 months at 60 °C are shown in Table 5.

**[Table 5]**

| | Comparative Example 1 | Comparative Example 2 | Comparative Example 3 | Example 12 | Example 14 | Example 15 |
|---|---|---|---|---|---|---|
| Related Substance 1 | 0.20% | 7.34% | 0.15% | 0.55% | 0.28% | 0.89% |
| Related Substance 2 | 5.75% | 4.48% | 5.17% | 1.01% | 1.00% | 1.88% |
| Related Substance 3 | 0.41% | - | 0.13% | 0.09% | 0.05% | 0.07% |
| Related Substance 4 | 2.12% | - | - | - | - | - |

Compared to Examples 12, 14 and 15, a marked increase of the related substances production was noted in Comparative Example 1, 2 and 3. That is, together with a marked increase of Related substance 2, a specific related substance (Related substance 4), which was not observed in the other Examples, was produced in Comparative Example 1. Related substances 1 and 2 were remarkably produced in Comparative Example 2. Also related substance 2 was remarkably increased in Comparative Example 3. As observed above, it was confirmed that use of mannitol and anhydrous calcium hydrogen phosphate as an excipient as well as use of only crystalline cellulose as an excipient caused production of a large amount of related substances, and therefore, use of them as an excipient was not preferable. On the other hand, it was found that use of lactose as a main excipient allowed cariprazine hydrochloride to be chemically stably stored.

### Experimental Example 4: Stability Test

The tablets obtained in Example 18 and Example 20 were packaged in accordance with the method described in Experimental Example 1 to give the PTP packaged products, the PTP aluminum bag packaged products and the PTP silica gel aluminum bag packaged products. The stability test of each of the obtained packaged product was conducted by storing in a constant temperature and humidity room (the room temperature was 40 °C; the relative humidity was 75 %).
The storage period was 6 months. After taking out from the package, the water activity of the tablet was measured. Then, the related substances were confirmed by a high-performance liquid chromatography. The water activity and the amount of related substance of the tablet obtained in Example 18 are shown in Table 6. The water activity and the amount of related substance of the tablet obtained in Example 20 are shown in Table 7. AQUALAB (series3TE, DECAGON) was used as a water activity measurement system. The measurement of the water activity was conducted within 12 hours of taking out the tablet from the constant temperature and humidity room. In addition, the formulation and the method for preparation used in Example 18 are in accordance with those used in Example 14 except that the amount of the binder was increased from 2 % to 3 % of the total amount and the amount of cornstarch was decreased for offsetting the increase of the binder.

**[Table 6]**

| Related substances production under the storage period for 6 months of the tablet obtained in Example 18 | | | |
|---|---|---|---|
| Water Activity | 70.0% (PTP packaged product) | 21.8% (PTP aluminum bag packaged product) | 7.6% (PTP silica gel aluminum packaged product) |
| Related substance 1 | 0.40% | 0.75% | 0.44% |
| Related substance 2 | 0.04% | 0.47% | 0.83% |
| Related substance 3 | 0.13% | 0.18% | 0.14% |
| Total Amount | 0.57% | 1.40% | 1.41% |

**[Table 7]**

| Related substances production under the storage period for 6 months of the tablet obtained in Example 20 | | | |
|---|---|---|---|
| Water Activity | 70.0% (PTP packaged product) | 33.9% (PTP aluminum bag packaged product) | 7.8% (PTP silica gel aluminum packaged product) |
| Related substance 1 | 0.30% | 0.67% | 0.36% |
| Related substance 2 | 0.05% | 0.08% | 0.35% |
| Related substance 3 | 0.05% | 0.17% | 0.17% |
| Total Amount | 0.40% | 0.92% | 0.88% |

As shown in Tables 6 and 7, it was confirmed that the tablet obtained in Example 20 had more excellent stability than the tablet obtained in Example 18. In particular, the tablet of Example 20 was specifically stable under the low humidity condition and had less-dependency on the humidity. That is, it was found that the tablet which did not contain starch as the excipient was more excellent than the others.

### Experimental Example 5: Stability Test

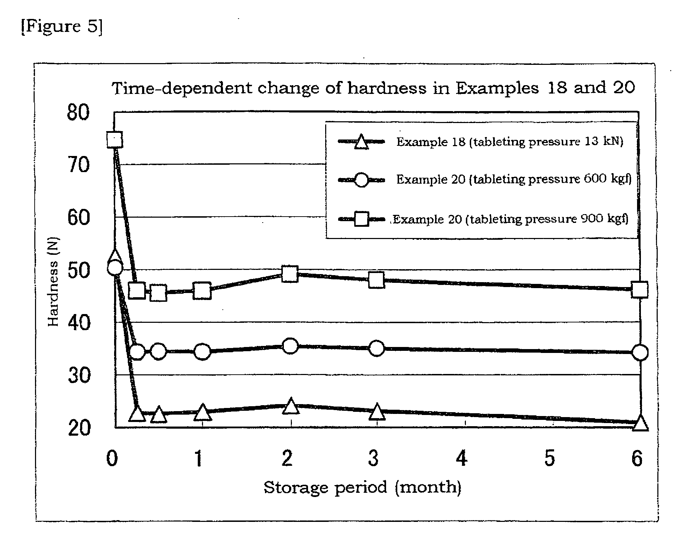

The stability tests of the PTP packaged products used in Experimental Example 4 (Example 18 and Example 20) were conducted by storing in a constant temperature and humidity room (the room temperature was 40 °C; the relative humidity was 75 %). The storage periods were 0.25, 0.5, 1, 2, 3 and 6 months. After taking out from the package, hardness was measured and the time-dependent change of the hardness was observed (Fig. 5).
As shown in Fig. 5, hardness of the tablet in Example 18 was remarkably reduced. Because desirable hardness which does not cause any problem for handling is normally more than 20 N, said tablet might have a storage problem under the high-humidity circumstance. On the other hand, hardness of the tablet of Example 20 remained within the allowable range. As observed above, it was found that the tablet which did not contain starch as an excipient was more excellent than the others. In addition, it was found that the tablet of Example 20 having adequate hardness could be prepared by compacting using a lower tableting pressure, and therefore, had excellent manufacturability.

### INDUSTRIAL APPLICABILITY

The present invention provides a solid preparation for oral administration wherein cariprazine hydrochloride can be stably stored without adding cyclodextrin.

## Claims

1. A solid preparation for oral administration which uses lactose as a main excipient and comprises cariprazine hydrochloride and which does not contain cyclodextrin.

2. The solid preparation according to claim 1 wherein an excipient other than lactose is crystalline cellulose and/or starch.

3. The solid preparation according to claim 2 wherein with respect to total amount of excipient, 50-100 % by weight is lactose, 0-50 % by weight is crystalline cellulose and 0-35 % by weight is starch.

4. The solid preparation according to claim 2 wherein with respect to total amount of excipient, 60-100 % by weight is lactose, 0-29 % by weight is crystalline cellulose and 0-11 % by weight is starch.

5. The solid preparation according to claim 2 wherein with respect to total amount of excipient, 70-100 % by weight is lactose, 0-25 % by weight is crystalline cellulose and 0-5 % by weight is starch.

6. The solid preparation according to claim 2 wherein with respect to total amount of excipient, 80-95 % by weight is lactose, 5-20 % by weight is crystalline cellulose and no starch is contained in the solid preparation.

7. The solid preparation according to any one of claims 1-6 wherein at least one binder selected from hydroxypropyl cellulose, hydroxypropyl methylcellulose, ethylcellulose, methylcellulose, povidone and polyvinyl alcohol is used.

8. The solid preparation according to any one of claims 1-7 wherein at least one disintegrant selected from sodium starch glycolate, croscarmellose sodium, low substituted hydroxypropyl cellulose, powdered agar and crospovidone is used.

9. The solid preparation according to any one of claims 1-8, wherein the preparation is prepared by blending cariprazine hydrochloride, an excipient, a binder, and, if needed, a disintegrant; granulating the mixture; blending a disintegrant and a lubricant to the granule; and then compacting the granule.

## Patentansprüche

1. Feste Zubereitung für die orale Verabreichung, welche Lactose als Haupthilfsstoff enthält und Cariprazinhydrochlorid umfasst und welche kein Cyclodextrin enthält.

2. Feste Zubereitung gemäß Anspruch 1, worin ein anderer Hilfsstoff als Lactose kristalline Cellulose und/oder Stärke ist.

3. Feste Zubereitung gemäß Anspruch 2, worin bezogen auf die gesamte Hilfsstoffmenge 50 bis 100 Gew.% Lactose ist, 0 bis 50 Gew.% kristalline Cellulose ist und 0 bis 35 Gew.% Stärke ist.

4. Feste Zubereitung gemäß Anspruch 2, worin bezogen auf die gesamte Hilfsstoffmenge 60 bis 100 Gew.% Lactose ist, 0 bis 29 Gew.% kristalline Cellulose ist und 0 bis 11 Gew.% Stärke ist.

5. Feste Zubereitung gemäß Anspruch 2, worin bezogen auf die gesamte Hilfsstoffmenge 70 bis 100 Gew.% Lactose ist, 0 bis 25 Gew.% kristalline Cellulose ist und 0 bis 5 Gew.% Stärke ist.

6. Feste Zubereitung gemäß Anspruch 2, worin bezogen auf die gesamte Hilfsstoffmenge 80 bis 95 Gew.% Lactose ist, 5 bis 20 Gew.% kristalline Cellulose ist und keine Stärke in der festen Zubereitung enthalten ist.

7. Feste Zubereitung gemäß irgendeinem der Ansprüche 1 bis 6, worin mindestens ein Bindemittel ausgewählt aus Hydroxypropylcellulose, Hydroxypropylmethylcellulose, Ethylcellulose, Methylcellulose, Povidon und Polyvinylalkohol, verwendet wird.

8. Feste Zubereitung gemäß irgendeinem der Ansprüche 1 bis 7, worin mindestens ein Sprengmittel verwendet wird, das aus Natriumstärkeglycolat, Croscarmellosenatrium, niedrig-substituierter Hydroxypropylcellulose, pulverisiertem Agar und Crospovidon ausgewählt ist.

9. Feste Zubereitung gemäß irgendeinem der Ansprüche 1 bis 8, wobei die Zubereitung hergestellt wird durch Mischen von Cariprazinhydrochlorid, einem Hilfsstoff, einem Bindemittel, und, falls erforderlich, einem Sprengmittel; Granulieren der Mischung; Beimischen eines Sprengmittels und eines Gleitmittels zu dem Granulat; und dann Verdichten des Granulats.

## Revendications

1. Préparation solide pour administration orale qui utilise du lactose en tant que principal excipient et qui comprend du chlorhydrate de cariprazine et qui ne contient pas de cyclodextrine.

2. Préparation solide selon la revendication 1, dans laquelle un excipient autre que le lactose est de la cellulose cristalline et/ou de l'amidon.

3. Préparation solide selon la revendication 2, dans laquelle par rapport à la quantité totale d'excipient, de 50 à 100 % en poids sont constitués de lactose, de 0 à 50 % en poids sont constitués de cellulose cristalline et de 0 à 35 % en poids sont constitués d'amidon.

4. Préparation solide selon la revendication 2, dans laquelle par rapport à la quantité totale d'excipient, de 60 à 100 % en poids sont constitués de lactose, de 0 à 29 % en poids sont constitués de cellulose cristalline et de 0 à 11 % en poids sont constitués d'amidon.

5. Préparation solide selon la revendication 2, dans laquelle par rapport à la quantité totale d'excipient, de 70 à 100 % en poids sont constitués de lactose, de 0 à 25 % en poids sont constitués de cellulose cristalline et de 0 à 5 % en poids sont constitués d'amidon.

6. Préparation solide selon la revendication 2, dans laquelle par rapport à la quantité totale d'excipient, de 80 à 95 % en poids sont constitués de lactose, de 5 à 20 % en poids sont constitués de cellulose cristalline, et une quantité nulle d'amidon est présente dans la préparation solide.

7. Préparation solide selon l'une quelconque des revendications 1 à 6, dans laquelle au moins un liant choisi parmi l'hydroxypropylcellulose, l'hydroxypropylméthylcellulose, l'éthylcellulose, la méthylcellulose, la povidone et l'alcool polyvinylique est utilisé.

8. Préparation solide selon l'une quelconque des revendications 1 à 7, dans laquelle au moins un désintégrant choisi parmi le glycolate d'amidon sodique, la croscarmellose sodique, l'hydroxypropylcellulose à bas degré de substitution, l'agar en poudre et la crospovidone est utilisé.

9. Préparation solide selon l'une quelconque des revendications 1 à 8, dans laquelle la préparation est préparée par mélange de chlorhydrate de cariprazine, d'un excipient, d'un liant, et, si nécessaire, d'un désintégrant ; granulation du mélange ; mélange d'un désintégrant et d'un lubrifiant avec le granule ; puis compaction du granule.
